# EUROPEAN PATENT APPLICATION

(11) **EP 4 752 164 A1**
(43) Date of publication of application: **03.06.2026**
(21) Application number: 24887221.0
(22) Date of filing: 24.01.2024
(51) Int. Cl.: C08G 18/66, C08G 18/42, C08G 18/34, C08G 18/32, C08G 18/44, C08G 18/48, A41D 19/00, A61F 6/04

(54) **POLYURETHANE-POLYUREA AQUEOUS DISPERSION, AND PREPARATION METHOD THEREFOR AND USE THEREOF**

(30) Priority: 09.11.2023 CN 202311482462
(71) Applicant: Wanhua Chemical Group Co., Ltd., Yantai, Shandong 264006 (CN)
(72) Inventor: WANG, Zhen, Yantai, Shandong 264006 (CN); JIN, Yunquan, Yantai, Shandong 264006 (CN); ZHANG, Xu, Yantai, Shandong 264006 (CN); LIU, Yiming, Yantai, Shandong 264006 (CN); JI, Xueshun, Yantai, Shandong 264006 (CN); SUN, Jiakuan, Yantai, Shandong 264006 (CN)
(74) Representative: Potter Clarkson
(86) International application number: PCT/CN2024/073749
(87) International publication number: WO 2025/097592

(57) **Abstract**

Disclosed are a polyurethane-polyurea aqueous dispersion, and a preparation method therefor and a use thereof. The aqueous dispersion comprises a polyurethane-polyurea dispersed therein, containing a terminal carboxyl group, a sulfonate group, and a carboxylate group, and having a specific theoretical molecular weight, wherein the content of the carboxylate group is 1.49-11.9 mmol/100 g, the content of the sulfonate group is 0.26-7.9 mmol/100 g, the content of the terminal carboxyl group is 0.5-3.7 mmol/100 g, the molecular weight is 60,000-390,000, and the specific theoretical molecular weight is a theoretical design molecular weight and is obtained by means of an end-group calculation method. The polyurethane-polyurea aqueous dispersion avoids the problems of glove cracking and pinholes in glove production and improves production stability.

## Description

### TECHNICAL FIELD

The present application relates to the field of polyurethane-polyurea dispersions, and in particular to a polyurethane-polyurea aqueous dispersion, a method for its preparation and use thereof.

### BACKGROUND

Currently, research on polyurethane-polyurea aqueous dispersions for disposable polyurethane gloves is limited, and no industrialized glove products are commercially available. Studies have revealed that the key challenge lies in the fact that the stability of the dispersion directly impacts the pass rate of gloves, making dispersion stability a critical factor in glove production.

Most existing solutions focus on the fundamental physical properties of polyurethane gloves but fail to address issues concerning pass rate, which is precisely the key factor hindering the industrial-scale production of the gloves.

There is an urgent need in this field to address the problem concerning pass rate by eliminating defects such as glove cracking and pinholes during glove production, thereby improving the pass rate in mass production.

### SUMMARY OF THE INVENTION

One object of the present application is to provide a polyurethane-polyurea aqueous dispersion. Through research on factors influencing flocculation, such as the type and content of ionic groups and molecular weight design, the inventors have developed a low-defect dispersion suitable for stable industrial-scale production. This ensures that the products remain free from cracking and pinholes during the manufacturing process, thereby significantly improving the pass rate.

To achieve the above object, the present application adopts the following technical solution:
A polyurethane-polyurea aqueous dispersion, including a polyurethane-polyurea dispersed therein, wherein the polyurethane-polyurea contains terminal carboxyl groups, sulfonate groups, and carboxylate groups, and has a specific theoretical molecular weight, wherein the carboxylate groups have a content of 1.49-11.9 mmol/100 g, the sulfonate groups have a content of 0.26-7.9 mmol/100 g, the terminal carboxyl groups have a content of 0.5-3.7 mmol/100g, and the molecular weight is from 60000 to 390000, and wherein the specific theoretical molecular weight is a theoretically designed molecular weight obtained by an end-group calculation method.

In one embodiment of the present application, the dispersion is prepared by reacting raw materials containing the following components:
component a: a macromolecular polyol;
component b: a polyisocyanate;
component c: a hydrophilic compound including component c1 and component c2; wherein component c1 contains 2-3 hydroxyl groups reactive with NCO and a carboxyl group functioning as a hydrophilic group; and component c2 contains 2-3 amino groups reactive with NCO and a salt-forming group functioning as a hydrophilic group;
optionally, component d: a low molecular weight alcohol chain extender;
component e: a compound for ionizing latent groups of component c;
component f: a polyamine having a number-average molecular weight of not more than 500 g/mol; and
component g: a mono-functional active compound containing a carboxyl group and further containing an amino group capable of reacting with isocyanates.

In one embodiment of the present application, the components of the dispersion are present in the following proportions:
component a: 65-90 parts, preferably 74-88 parts;
component b: 8-25 parts, preferably 10-20 parts;
component c: 1-6 parts, preferably 1.5-4.8 parts;
component d: 0-4 parts, preferably 0-3 parts;
component e: 0.1-5 parts, preferably 0.1-3 parts;
component f: 0-6 parts, preferably 0-3 parts; and
component g: 0.05-1 part, preferably 0.085-0.6 part. The proportions of said components are based on mass.

In the present application, a mono-functional compound g containing a carboxyl group is reacted with isocyanates to form a polyurethane segment having terminal carboxyl groups.

The introduction of the mono-functional compound enables control of the molecular weight of the product within a suitable range, thereby avoiding production defects in glove products caused by inappropriate molecular weight. Furthermore, the terminal carboxyl groups are more reactive than the carboxyl groups in the chain. When the dispersion is used to prepare articles such as gloves, the reaction efficiency between the terminal carboxyl groups of the dispersion and the etherified amino resin used as the base resin for gloves is enhanced, significantly increasing crosslinking degree and preventing a reduction in the mechanical properties of the gloves due to end-capping.

In the polyurethane-polyurea dispersion provided in the present application, the combined use of hydrophilic groups achieves a balance of electrolyte sensitivity of the emulsion: an appropriate content of carboxylate ions imparts electrolyte sensitivity to the dispersion, while an appropriate content of sulfonate ions exhibits weaker electrolyte sensitivity than the carboxylate ions. This reduces the occurrence of slagging and skinning during glove preparation. By controlling the molecular weight of the polyurethane within a suitable range, the wet film formed by instant demulsification of glue solution will not crack due to volume shrinkage resulting from high molecular weight and large cohesive energy, nor will it slip due to insufficient strength resulting from low molecular weight, thereby preventing issues such as cracking and glue sliding.

In one embodiment of the present application, the component a is one or more selected from the group consisting of a diol, a triol, and a tetraol, each having a number-average molecular weight of 500-6000 g/mol; and preferably one or more selected from the group consisting of a polyester polyol, a polyether polyol, a polycarbonate polyol, and a polycaprolactone polyol, each having a functionality of 2-3 and a number-average molecular weight of 500-5000 g/mol.

In one embodiment of the present application, the component b is one or more selected from the group consisting of an aromatic polyisocyanate, an aliphatic polyisocyanate, and an alicyclic polyisocyanate, and preferably one or more selected from the group consisting of an aromatic polyisocyanate, an aliphatic polyisocyanate, and an alicyclic polyisocyanate each have at least two isocyanate groups.

The component b is preferably represented by the molecular formula Y(NCO)₂, where Y represents a divalent aliphatic hydrocarbon group containing 4-12 carbon atoms, a divalent alicyclic hydrocarbon group containing 6-15 carbon atoms, a divalent aromatic hydrocarbon group containing 6-15 carbon atoms, or a divalent araliphatic hydrocarbon group containing 7-15 carbon atoms. Suitable diisocyanates include tetramethylene diisocyanate, methylpentamethylene diisocyanate, hexamethylene diisocyanate, dodecamethylene diisocyanate, 1,4-cyclohexane diisocyanate, isophorone diisocyanate, 4,4'-dicyclohexylmethane diisocyanate, 4,4'-dicyclohexylpropane diisocyanate, 1,4-phenylene diisocyanate, 2,4-toluene diisocyanate, 2,6-toluene diisocyanate, 4,4'-diphenylmethane diisocyanate, 2,2'-diphenylmethane diisocyanate and 2,4'-diphenylmethane diisocyanate, tetramethylxylylene diisocyanate, p-xylylene diisocyanate, p-isopropylidene diisocyanate, and mixtures composed of these compounds.

The component b may also include a small amount of higher functionality polyisocyanates known in polyurethane chemistry or modified polyisocyanates containing groups such as carbodiimide group, allophanate group, isocyanurate group, carbamate group, and/or biuret group. Preferred polyisocyanates are hexamethylene diisocyanate, isophorone diisocyanate, dicyclohexylmethane diisocyanate, diphenylmethane diisocyanate, and toluene diisocyanate.

In one embodiment of the present application, in the component c, component c1 is dimethylolpropionic acid and/or dimethylolbutanoic acid, and component c2 is sodium 2-[(2-aminoethyl)amino]ethanesulphonate and/or sodium ethylenediamine propionate.

In one embodiment of the present application, the component d is one or more selected from the group consisting of ethylene glycol, 1,2-propanediol, 1,3-propanediol, 1,4-butanediol, 1,3-butanediol, 2,3-butanediol, 1,5-pentanediol, 1,6-hexanediol, neopentyl glycol, 1,4-cyclohexanediol, 1,4-bis(hydroxymethyl)cyclohexane, 1,8-octanediol, 1,10-decanediol, 1,12-dodecanediol, 1,4-cyclohexanediol, 1,4-cyclohexanedimethanol, and 2-ethyl-1,3-hexanediol; and preferably one or more selected from the group consisting of 1,2-propanediol, 1,3-propanediol, 1,4-butanediol, 1,3-butanediol, neopentyl glycol, and 1,4-cyclohexanedimethanol.

In one embodiment of the present application, the component e is an organic base, preferably one or more selected from the group consisting of triethylamine, dimethylethanolamine, and aqueous ammonia, and more preferably triethylamine.

In one embodiment of the present application, the component f is a polyamine having a number-average molecular weight of 60-500 g/mol, preferably one or more selected from the group consisting of ethylenediamine, 1,2-diaminopropane, 1,4-diaminobutane, 1,6-hexanediamine, 2-methyl-1,5-pentanediamine, isophorone diamine, 4,4'-diaminodicyclohexylmethane, piperazine, and diethylenetriamine, and more preferably ethylenediamine and/or isophorone diamine.

In one embodiment of the present application, said component g is a basic compound containing a carboxyl group capable of reacting with an etherified amino resin; preferably, the component g is a basic amino acid, such as histidine or phenylalanine, and more preferably phenylalanine.

Another object of the present application is to provide a method for preparing the polyurethane-polyurea aqueous dispersion.

A method for preparing the polyurethane-polyurea aqueous dispersion as mentioned above, including the following steps:
S1: charging component a, component b, component c1, optionally component d, and a solvent into a reactor, and reacting until an NCO content approaches or reaches a theoretical value to obtain an isocyanate-terminated prepolymer;
S2: cooling, and diluting with an additional amount of the solvent;
S3: adding component f and component c2 and allowing a reaction to proceed, followed by neutralization using component e, then adding component g and allowing a resulting mixture to react, dispersing a resulting reaction product with water to form a dispersion, and removing the solvent to obtain the polyurethane-polyurea aqueous dispersion free of the solvent.

In one embodiment of the present application, the components used in the method are as follows:
component a: a macromolecular polyol;
component b: a polyisocyanate;
component c: a hydrophilic compound including component c1 and component c2; wherein component c1 contains 2-3 hydroxyl groups reactive with NCO and a carboxyl group functioning as a hydrophilic group; and component c2 contains 2-3 amino groups reactive with NCO and a salt-forming group functioning as a hydrophilic group;
optionally, component d: a low molecular weight alcohol chain extender;
component e: a compound for ionizing latent groups of component c;
component f: a polyamine having a number-average molecular weight of not more than 500 g/mol; and
component g: a mono-functional active compound containing a carboxyl group and further containing an amino group capable of reacting with isocyanates.

In one embodiment of the present application, the proportions of the components in the method are as follows:
component a: 65-90 parts, preferably 74-88 parts;
component b: 8-25 parts, preferably 10-20 parts;
component c: 1-6 parts, preferably 1.5-4.8 parts;
component d: 0-4 parts, preferably 0-3 parts;
component e: 0.1-5 parts, preferably 0.1-3 parts;
component f: 0-6 parts, preferably 0-3 parts; and
component g: 0.05-1 part, preferably 0.085-0.6 part.

In one embodiment of the present application, in step S1, the reaction is performed at a temperature of 60-80°C.

In one embodiment of the present application, the solvent in step S1 is acetone.

In one embodiment of the present application, in step S2, the heating reaction is performed at a temperature of 40-45°C.

In one embodiment of the present application, the solvent in step S2 is acetone.

In one embodiment of the present application, in step S3, component g is added after component c1 in the polyurethane is neutralized by component e and after the reaction of component c2 is completed.

In one embodiment of the present application, the heating reaction in step S3 is performed at a temperature of 40-45°C.

In one embodiment of the present application, the solvent in step S3 is acetone.

In one embodiment of the present application, the dispersion obtained in step S3 has a solid content of not less than 35 wt%.

Compared with the prior art, in step S3, component g is added after the neutralizing agent (component e) has been consumed. The purpose is not to enhance the hydrophilicity of the segment through neutralization, but to control the molecular weight via the mono-amino functionality. Furthermore, the presence of the carboxyl group enables crosslinking with the etherified amino resin used in subsequent glove preparation, and the acidity of the carboxyl group can also catalyze the crosslinking reaction.

Yet another object of the present application is to provide use of the polyurethane-polyurea aqueous dispersion.

Use of the polyurethane-polyurea aqueous dispersion, wherein the dispersion is the aforementioned dispersion or is prepared by the aforementioned method, and the use includes the preparation of gloves and/or condoms.

A further object of the present application is to provide a soft and elastic article.

A soft and elastic article, wherein the soft and elastic article is prepared from the aforementioned dispersion or from the dispersion prepared by the aforementioned method, and the soft and elastic article includes gloves and/or condoms.

Compared with the prior art, the technical solutions provided in the present application have the following beneficial effects:
In the polyurethane-polyurea dispersion provided in the present application, the combined use of hydrophilic groups achieves a balance of electrolyte sensitivity of the emulsion: carboxylate ions imparts electrolyte sensitivity, while sulfonate ions exhibits weaker electrolyte sensitivity than the carboxylate ions. This reduces the occurrence of slagging and skinning during glove preparation. By controlling the molecular weight of the polyurethane within a suitable range, the wet film formed by instant demulsification of glue solution will not crack due to volume shrinkage resulting from high molecular weight and large cohesive energy, nor will it slip due to insufficient strength resulting from low molecular weight, thereby preventing issues such as cracking and glue sliding.

### DETAILED DESCRIPTION

To facilitate understanding of the present application, the following will further illustrate the present application with reference to the examples. It should be understood that the following examples are only for better understanding of the present application and do not mean that the application is limited to the following examples.

Unless otherwise defined, all technical and scientific terms used herein have the same meaning as commonly understood by those skilled in the art to which the present application pertains. The term "and/or" used herein includes any and all combinations of one or more of the listed items.

For experimental steps or conditions not specified in the examples, conventional experimental procedures or conditions in the relevant technical field may be employed. Reagents or instruments for which the manufacturer is not specified are all commercially available conventional products.
Hexamethylene diisocyanate (HDI), isophorone diisocyanate (IPDI), and toluene diisocyanate (TDI): obtained from Wanhua Chemical;
Polyester polyol 1: polyadipic acid-neopentyl glycol polyester, Mn=2000, obtained from Wanhua Chemical;
Polyester polyol 2: polyadipic acid-isophthalic acid-1,4-butanediol polyester, Mn=2000, obtained from Wanhua Chemical;
Polyester polyol 3: polyadipic acid-2-methyl-1,3-propanediol polyester, Mn=2000, obtained from Wanhua Chemical;
Polyester polyol 4: polycarbonate polyol (initiator: 1,6-hexanediol and 1,5-pentanediol) Mn=2000, obtained from Asahi Kasei Corporation, Japan;
Polyester polyol 5: polyadipic acid-neopentyl glycol-hexanediol polyester, Mn=1500, obtained from Wanhua Chemical;
Polyether polyol 1: polyoxypropylene glycol, Mn=2000, functionality = 2, obtained from Wanhua Chemical;
Polyether polyol 2: polytetrahydrofuran glycol, Mn=2000, functionality = 2, obtained from Mitsubishi Chemical, Japan;
Dimethylolpropionic acid (DMPA): obtained from Perstorp;
Sodium 2-[(2-aminoethyl)amino]ethanesulphonate (50 wt% content), neopentyl glycol (NPG), and isophorone diamine (IPDA): obtained from Wanhua Chemical;
Triethylamine (TEA), phenylalanine, histidine, and ethanolamine: obtained from Sinopharm Chemical Reagent Co., Ltd.
pH value: Measured using a Metrohm 6173 pH meter.
Average particle size: The polyurethane dispersion was diluted with water to a concentration of 0.5 wt%, and measured using a Malvern Nano-ZS90.
   - NCO Determination: The -NCO content during polyurethane synthesis was determined according to the Chinese Chemical Industry Standard "HG/T2409-92 Determination of Isocyanate Group Content in Polyurethane Prepolymer". An NCO tester from Metrohm, Switzerland was used.

The specific theoretical molecular weight is a theoretically designed molecular weight, which is obtained based on the NCO end-group calculation method using the following formula: Theoretical Design Molecular Weight = 8400 / NCO% of the capped prepolymer.

### Example 1

400 g of polyester polyol 1, 78 g of IPDI, 7.0 g of DMPA, and 152 g of acetone were added to a 1 L four-necked round-bottom flask equipped with nitrogen inlet and outlet. The mixture was stirred at 80°C until the NCO content reached 1.2 wt% (theoretical value: 1.2 wt%). The temperature was lowered to 45°C, and 600 g of acetone was added to dilute the mixture while maintaining the temperature at 43°C. Then, under mechanical stirring, an aqueous mixture of 5 g of sodium 2-[(2-aminoethyl)amino]ethanesulphonate (50 wt% content) and 6 g of IPDA was added, and the reaction proceeded for 15 minutes. After addition of 6.0 g of triethylamine, neutralization was carried out for 5 minutes. 2.5 g of phenylalanine pre-diluted with 30 times its weight of water at 80°C was added, and the reaction was continued for 5 minutes. Under high-speed stirring at 1000 r/min, 950 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 35 wt%, an average particle size of 103 nm, and a pH value of 8.1. The theoretical design molecular weight was 65000 (sixty-five thousand). The terminal carboxyl group content was 2.9 mmol/100g, the carboxylate group content was 11.8 mmol/100g, and the sulfonate group content was 2.6 mmol/100g.

### Example 2

450 g of polyester polyol 2, 78 g of IPDI, 4.0 g of DMPA, and 152 g of acetone were added to a 1 L four-necked round-bottom flask equipped with nitrogen inlet and outlet. The mixture was stirred at 78°C until the NCO content reached 1.18 wt% (theoretical value: 1.18 wt%).

The temperature was lowered to 44°C, and 730 g of acetone was added to dilute the mixture while maintaining the temperature at 44°C. Then, under mechanical stirring, an aqueous mixture of 8 g of sodium 2-[(2-aminoethyl)amino]ethanesulphonate (50 wt% content) and 5 g of IPDA was added, and the reaction proceeded for 15 minutes. After addition of 3.0 g of triethylamine, neutralization was carried out for 5 minutes. 0.8 g of phenylalanine pre-diluted with 30 times its weight of water at 80°C was added, and the reaction was continued for 5 minutes. Under high-speed stirring at 1000 r/min, 1098 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 35 wt%, an average particle size of 173 nm, and a pH value of 8.5. The theoretical design molecular weight was 223000. The terminal carboxyl group content was 0.87 mmol/100g, the carboxylate group content was 5.4 mmol/100g, and the sulfonate group content was 3.8 mmol/100g.

### Example 3

450 g of polyester polyol 3, 45 g of HDI, 42 g of IPDI, 2.0 g of DMPA, 8 g of NPG, and 170 g of acetone were added to a 1 L four-necked round-bottom flask equipped with nitrogen inlet and outlet. The mixture was stirred at 75°C until the NCO content reached 1.64 wt% (theoretical value: 1.64 wt%). The temperature was lowered to 43°C, and 700 g of acetone was added to dilute the mixture while maintaining the temperature at 45°C. Then, under mechanical stirring, an aqueous mixture of 15 g of sodium 2-[(2-aminoethyl)amino]ethanesulphonate (50 wt% content) and 5 g of IPDA was added, and the reaction proceeded for 15 minutes. After addition of 1.2 g of triethylamine, neutralization was carried out for 5 minutes. 1.5 g of phenylalanine pre-diluted with 30 times its weight of water at 80°C was added, and the reaction was continued for 5 minutes. Under high-speed stirring at 1000 r/min, 1130 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 37 wt%, an average particle size of 115 nm, and a pH value of 8.3. The theoretical design molecular weight was 123000. The terminal carboxyl group content was 1.59 mmol/100g, the carboxylate group content was 2.62 mmol/100g, and the sulfonate group content was 3.69 mmol/100g.

### Example 4

560 g of polyester polyol 4, 45 g of HDI, 48 g of IPDI, 15 g of DMPA, and 185 g of acetone were added to a 1 L four-necked round-bottom flask equipped with nitrogen inlet and outlet. The mixture was stirred at 80°C until the NCO content reached 0.9 wt% (theoretical value: 0.9 wt%). The temperature was lowered to 45°C, and 700 g of acetone was added to dilute the mixture while maintaining the temperature at 43°C. Then, under mechanical stirring, 5 g of sodium 2-[(2-aminoethyl)amino]ethanesulphonate (50 wt% content) pre-diluted with water was added, and the reaction proceeded for 15 minutes. After addition of 5.66 g of triethylamine, neutralization was carried out for 5 minutes. 1.0 g of histidine pre-diluted with 30 times its weight of water at 80°C was added, and the reaction was continued for 5 minutes. Then, under high-speed stirring at 1000 r/min, 1330 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 35 wt%, an average particle size of 90 nm, and a pH value of 8.3. The theoretical design molecular weight was 221000. The terminal carboxyl group content was 0.9 mmol/100g, the carboxylate group content was 8.2 mmol/100g, and the sulfonate group content was 1.9 mmol/100g.

### Example 5

300 g of polyether polyol 1, 300 g of polyether polyol 2, 25 g of TDI, 90 g of IPDI, 19 g of DMPA, and 185 g of acetone were added to a 1 L four-necked round-bottom flask equipped with nitrogen inlet and outlet. The mixture was stirred at 80°C until the NCO content reached 0.97 wt% (theoretical value: 0.97 wt%). The temperature was lowered to 45°C, and 700 g of acetone was added to dilute the mixture while maintaining the temperature at 43°C. Then, under mechanical stirring, an aqueous mixture of 9 g of sodium 2-[(2-aminoethyl)amino]ethanesulphonate (50 wt% content) and 3 g of IPDA was added, and the reaction proceeded for 15 minutes. After addition of 7.17 g of triethylamine, neutralization was carried out for 5 minutes. 0.70 g of phenylalanine pre-diluted with 30 times its weight of water at 80°C was added, and the reaction was continued for 5 minutes. Then, under high-speed stirring at 1000 r/min, 1530 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 35 wt%, an average particle size of 110 nm, and a pH value of 8.7. The theoretical design molecular weight was 350000. The terminal carboxyl group content was 0.56 mmol/100g, the carboxylate group content was 9.4 mmol/100g, and the sulfonate group content was 3.1 mmol/100g.

### Example 6

450 g of polyester polyol 5, 25 g of TDI, 90 g of IPDI, 19 g of DMPA, and 185 g of acetone were added to a 1 L four-necked round-bottom flask equipped with nitrogen inlet and outlet. The mixture was stirred at 80°C until the NCO content reached 1.14 wt% (theoretical value: 1.14 wt%). The temperature was lowered to 45°C, and 700 g of acetone was added to dilute the mixture while maintaining the temperature at 43°C. Then, under mechanical stirring, an aqueous mixture of 9 g of sodium 2-[(2-aminoethyl)amino]ethanesulphonate (50 wt% content) and 3 g of IPDA was added, and the reaction proceeded for 15 minutes. After addition of 7.17 g of triethylamine, neutralization was carried out for 5 minutes. 0.70 g of phenylalanine pre-diluted with 30 times its weight of water at 80°C was added, and the reaction was continued for 5 minutes. Then, under high-speed stirring at 1000 r/min, 1230 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 38 wt%, an average particle size of 100 nm, and a pH value of 8.7. The theoretical design molecular weight was 280000. The terminal carboxyl group content was 0.69 mmol/100g, the carboxylate group content was 11.75 mmol/100g, and the sulfonate group content was 3.93 mmol/100g.

### Comparative Example 1

It differed from Example 2 in that 0.8 g of phenylalanine was replaced with an equimolar amount of ethanolamine (0.3 g), which does not contain a carboxyl group.

The dispersion exhibited a solid content of 35 wt%, an average particle size of 178 nm, and a pH value of 8.5. The theoretical design molecular weight was 223000, the terminal carboxyl group content was 0 mmol/100g, the carboxylate group content was 5.4 mmol/100g, and the sulfonate group content was 3.8 mmol/100g.

### Comparative Example 2

It differed from Example 1 in that component g was added before neutralization.

400 g of polyester polyol 1, 78 g of IPDI, 7.0 g of DMPA, and 152 g of acetone were added to a 1 L four-necked round-bottom flask equipped with nitrogen inlet and outlet. The mixture was stirred at 80°C until the NCO content reached 1.2 wt% (theoretical value: 1.2 wt%). The temperature was lowered to 45°C, and 600 g of acetone was added to dilute the mixture while maintaining the temperature 43°C. Then, under mechanical stirring, an aqueous mixture of 5 g of sodium 2-[(2-aminoethyl)amino]ethanesulphonate (50 wt% content) and 6 g of IPDA was added, and the reaction proceeded for 15 minutes. 2.5 g of phenylalanine pre-diluted with 30 times its weight of water at 80°C was added, and the reaction was continued for 5 minutes. After addition of 7.3 g of triethylamine, neutralization was carried out for 5 minutes. Under high-speed stirring at 1000 r/min, 950 g of deionized water was added, and the mixture was dispersed for 15 minutes. After removing acetone via vacuum distillation at 55°C and a vacuum degree of 0.09 MPa, a solvent-free dispersion was obtained, which exhibited a solid content of 35 wt%, an average particle size of 109 nm, and a pH value of 8.2. The theoretical design molecular weight was 65000. The terminal carboxyl group content was 0.42 mmol/100g, the carboxylate group content was 14.28 mmol/100g, and the sulfonate group content was 2.6 mmol/100g.

**Preparation of Base Resin (Stock Solution) for Glove:** The prepared dispersion was diluted with deionized water to a solid content of 15 wt%. Based on the mass of the diluted dispersion, 0.5 wt% color paste, 5 wt% titanium dioxide, and 1.5 % methylated amino resin were added, and the mixture was stirred at low speed (250 r/min) for 30 minutes and then kept for use.

**Preparation of Coagulant Solution:** An aqueous solution containing 10 wt% CaCl₂ and 5 wt% release agent (calcium stearate) was prepared using deionized water and stirred continuously for 1 h. The solution was then kept for use.

### Glove Preparation:

1) The cleaned glove mold was placed in an oven at 100°C and dried.
2) The dried glove mold was taken out and cooled to 55°C, immersed in the coagulant solution for 8 seconds, taken out, placed in an oven at 130°C and dried.
3) The dried glove mold was taken out, cooled to 60°C, immersed in the prepared base resin for 8 seconds, taken out, and left to stand for 1 minute (observing whether slippage occurred at the wrist area of the glove). They were then immersed in deionized water at 50°C for 1 minute, taken out, placed in an oven at 130°C and baked for 18 minutes.
4) After baking, the glove mold was taken out, immersed in a glove finishing agent, taken out, air-dried naturally, and demolded to obtain finished gloves.

### Performance Testing:

Cracking Test: After demolding, the gloves were inflated, and linear or cross-shaped cracks were observed.

### Tensile Strength Test:

The palm part of the glove was cut into a dumbbell-shaped specimen with a width of 6 mm and a length of 115 mm. The tensile strength was measured at a tensile rate of 200 mm/min using a tensile testing machine from GOTECH TESTING MACHINES CO., LTD.

Alcohol Resistance Test: The inflated gloves were sprayed with 55% concentration of alcohol on the surface, and dissolution-induced cracking at the finger crotches was observed.

The performance test results are shown in Table 1.

| | Cracking | Tensile Strength / MPa | Alcohol Resistance |
|---|---|---|---|
| Example 1 | None | 28 | No dissolution-induced cracking |
| Example 2 | None | 30 | No dissolution-induced cracking |
| Example 3 | None | 29 | No dissolution-induced cracking |
| Example 4 | None | 30 | No dissolution-induced cracking |
| Example 5 | None | 30 | No dissolution-induced cracking |
| Example 6 | None | 31 | No dissolution-induced cracking |
| Comparative Example 1 | None | 23 | Dissolution-induced cracking observed |
| Comparative Example 2 | None | 26 | Slight dissolution-induced cracking |

As can be seen from the table, gloves prepared from the polyurethane-polyurea aqueous dispersion according to the present application exhibit no cracking, with a high pass rate and excellent alcohol resistance.

It will be readily understood that the examples described above are provided for illustrative purposes only and are not intended to limit the scope of the present application. Those of ordinary skill in the art may make various modifications and variations based on the foregoing description. It is neither necessary nor feasible to exhaustively enumerate all possible embodiments. Any obvious modifications or variations derived from the disclosed content shall still fall within the scope of protection of the present application.

## Claims

1. A polyurethane-polyurea aqueous dispersion, comprising a polyurethane-polyurea dispersed therein, wherein the polyurethane-polyurea contains terminal carboxyl groups, sulfonate groups, and carboxylate groups, and has a specific theoretical molecular weight, wherein the carboxylate groups have a content of 1.49-11.9 mmol/100 g, the sulfonate groups have a content of 0.26-7.9 mmol/100 g, the terminal carboxyl groups have a content of 0.5-3.7 mmol/100g, and the molecular weight is from 60000 to 390000, and wherein the specific theoretical molecular weight is a theoretically designed molecular weight obtained by an end-group calculation method.

2. The dispersion according to claim 1, wherein the dispersion is prepared by reacting raw materials comprising the following components:
component a: a macromolecular polyol;
component b: a polyisocyanate;
component c: a hydrophilic compound comprising component c1 and component c2; wherein component c1 contains 2-3 hydroxyl groups reactive with NCO and a carboxyl group functioning as a hydrophilic group; and component c2 contains 2-3 amino groups reactive with NCO, and a salt-forming group functioning as a hydrophilic group;
optionally, component d: a low molecular weight alcohol chain extender;
component e: a compound for ionizing latent groups of component c;
component f: a polyamine having a number-average molecular weight of not more than 500 g/mol; and
component g: a mono-functional active compound containing a carboxyl group and further containing an amino group capable of reacting with isocyanates.

3. The dispersion according to claim 1 or 2, wherein the components of the dispersion are present in the following proportions:
component a: 65-90 parts, preferably 74-88 parts;
component b: 8-25 parts, preferably 10-20 parts;
component c: 1-6 parts, preferably 1.5-4.8 parts;
component d: 0-4 parts, preferably 0-3 parts;
component e: 0.1-5 parts, preferably 0.1-3 parts;
component f: 0-6 parts, preferably 0-3 parts; and
component g: 0.05-1 part, preferably 0.085-0.6 part.

4. The dispersion according to any one of claims 1 to 3, wherein the component a is one or more selected from the group consisting of a diol, a triol, and a tetraol, each having a number-average molecular weight of 500-6000 g/mol; and preferably one or more selected from the group consisting of a polyester polyol, a polyether polyol, a polycarbonate polyol, and a polycaprolactone polyol, each having a functionality of 2-3 and a number-average molecular weight of 500-5000 g/mol.

5. The dispersion according to any one of claims 1 to 4, wherein the component b is one or more selected from the group consisting of an aromatic polyisocyanate, an aliphatic polyisocyanate, and an alicyclic polyisocyanate, and preferably one or more selected from the group consisting of an aromatic polyisocyanate, an aliphatic polyisocyanate, and an alicyclic polyisocyanate each have at least two isocyanate groups.

6. The dispersion according to any one of claims 1 to 5, wherein in the component c, component c1 is dimethylolpropionic acid and/or dimethylolbutanoic acid, and component c2 is sodium 2-[(2-aminoethyl)amino]ethanesulphonate and/or sodium ethylenediamine propionate.

7. The dispersion according to any one of claims 1 to 6, wherein the component d is one or more selected from the group consisting of ethylene glycol, 1,2-propanediol, 1,3-propanediol, 1,4-butanediol, 1,3-butanediol, 2,3-butanediol, 1,5-pentanediol, 1,6-hexanediol, neopentyl glycol, 1,4-cyclohexanediol, 1,4-bis(hydroxymethyl)cyclohexane, 1,8-octanediol, 1,10-decanediol, 1,12-dodecanediol, 1,4-cyclohexanediol, 1,4-cyclohexanedimethanol, and 2-ethyl-1,3-hexanediol; preferably one or more selected from the group consisting of 1,2-propanediol, 1,3-propanediol, 1,4-butanediol, 1,3-butanediol, neopentyl glycol, and 1,4-cyclohexanedimethanol.

8. The dispersion according to any one of claims 1 to 7, wherein the component e is an organic base, preferably one or more selected from the group consisting of triethylamine, dimethylethanolamine, and aqueous ammonia, and more preferably triethylamine.

9. The dispersion according to any one of claims 1 to 8, wherein the component f is a polyamine having a number-average molecular weight of 60-500 g/mol, preferably one or more selected from the group consisting of ethylenediamine, 1,2-diaminopropane, 1,4-diaminobutane, 1,6-hexanediamine, 2-methyl-1,5-pentanediamine, isophorone diamine, 4,4'-diaminodicyclohexylmethane, piperazine, and diethylenetriamine, and more preferably ethylenediamine and/or isophorone diamine.

10. The dispersion according to any one of claims 1 to 9, wherein the component g is a basic compound containing a carboxyl group capable of reacting with an etherified amino resin; preferably, the component g is a basic amino acid, such as histidine or phenylalanine, and more preferably phenylalanine.

11. A method for preparing the polyurethane-polyurea aqueous dispersion according to any one of claims 1 to 10, comprising the following steps:
S1: charging component a, component b, component c1, optionally component d, and a solvent into a reactor, and reacting until an NCO content approaches or reaches a theoretical value to obtain an isocyanate-terminated prepolymer;
S2: cooling, and diluting with an additional amount of the solvent;
S3: adding component f and component c2 and allowing a reaction to proceed, followed by neutralization using component e, then adding component g and allowing a resulting mixture to react, dispersing a resulting reaction product with water to form a dispersion, and removing the solvent to obtain the polyurethane-polyurea aqueous dispersion free of the solvent.

12. The method according to claim 11, wherein the components used in the method are as follows:
component a: a macromolecular polyol;
component b: a polyisocyanate;
component c: a hydrophilic compound comprising component c1 and component c2; wherein component c1 contains 2-3 hydroxyl groups reactive with NCO and a carboxyl group functioning as a hydrophilic group; and component c2 contains 2-3 amino groups reactive with NCO and a salt-forming group functioning as a hydrophilic group;
optionally, component d: a low molecular weight alcohol chain extender;
component e: a compound for ionizing latent groups of component c;
component f: a polyamine having a number-average molecular weight of not more than 500 g/mol; and
component g: a mono-functional active compound containing a carboxyl group and further containing an amino group capable of reacting with isocyanates.

13. The method according to claim 11 or 12, wherein the proportions of the components in the method are as follows:
component a: 65-90 parts, preferably 74-88 parts;
component b: 8-25 parts, preferably 10-20 parts;
component c: 1-6 parts, preferably 1.5-4.8 parts;
component d: 0-4 parts, preferably 0-3 parts;
component e: 0.1-5 parts, preferably 0.1-3 parts;
component f: 0-6 parts, preferably 0-3 parts; and
component g: 0.05-1 part, preferably 0.085-0.6 part.

14. The method according to claim 11, wherein in step S1, the reaction is performed at a temperature of 60-80°C;
and/or the solvent in step S1 is acetone.

15. The method according to claim 11, wherein in step S2, the heating reaction is performed at a temperature of 40-45°C;
and/or the solvent in step S2 is acetone.

16. The method according to claim 11, wherein in step S3, component g is added after component c1 in the polyurethane is neutralized by component e and after the reaction of component c2 is completed;
and/or, the heating reaction in step S3 is performed at a temperature of 40-45°C;
and/or, the solvent in step S3 is acetone;
and/or, the dispersion obtained in step S3 has a solid content of not less than 35 wt%.

17. Use of the polyurethane-polyurea aqueous dispersion according to any one of claims 1-10 or the polyurethane-polyurea aqueous dispersion prepared by the method according to any one of claims 11-16, wherein the use comprises the preparation of gloves and/or condoms.

18. A soft and elastic article, wherein the soft and elastic article is prepared from the dispersion according to any one of claims 1-10 or from the dispersion prepared by the method according to any one of claims 11-16, wherein the soft and elastic article comprises gloves and/or condoms.
